# FASCICULE DE BREVET EUROPEEN

(11) **EP 3 568 462 B1**
(45) Date de publication et mention de la délivrance du brevet: **02.07.2025**
(21) Numéro de dépôt: 18703048.1
(22) Date de dépôt: 09.01.2018
(51) Int. Cl.: C12N 1/20, C12Q 1/04, C12N 1/38, C12R 1/32

(54) **MILIEU DE TRANSPORT ET/OU CONSERVATION POUR MYCOBACTERIUM TUBERCULOSIS**
TRANSPORT- UND/ODER SPEICHERMEDIUM FÜR MYCOBACTERIUM TUBERCULOSIS
TRANSPORT AND/OR STORAGE MEDIUM FOR MYCOBACTERIUM TUBERCULOSIS

(30) Priorité: 13.01.2017 FR 1750297
(43) Date de publication de la demande: 20.11.2019
(73) Titulaire: Fondation Méditerranée Infection, 13005 Marseille (FR); Université d'Aix-Marseille, 13007 Marseille (FR); Centre National de la Recherche Scientifique (CNRS), 75016 Paris (FR); Assistance Publique - Hôpitaux de Marseille, 13005 Marseille (FR); INSERM (Institut National de la Santé et de la Recherche Médicale), 75013 Paris (FR)
(72) Inventeur: RAOULT, Didier, 13008 Marseille (FR); DRANCOURT, Michel, 13012 Marseille (FR); NEAU, Elodie, 13002 Marseille (FR)
(74) Mandataire: Cabinet Beau de Loménie
(86) Numéro de dépôt international: PCT/FR2018/050045
(87) Numéro de publication internationale: WO 2018/130775

(56) Documents cités:
- WO-A1-02/45736
- WO-A1-2010/063911
- WO-A1-98/55624
- WO-A2-01/90309
- O. TURAPOV ET AL: "Antimicrobial Treatment Improves Mycobacterial Survival in Nonpermissive Growth Conditions", ANTIMICROBIAL AGENTS AND CHEMOTHERAPY, vol. 58, no. 5, 1 May 2014 (2014-05-01), pages 2798 - 2806, XP055456550, ISSN: 0066-4804, DOI: 10.1128/AAC.02774-13
- TOM H. JIN ET AL: "Identification of Growth Promoting Effect of <i>r</i>BCG/BCG Culture Supernatant and Its Potential Applications", WORLD JOURNAL OF VACCINES, vol. 03, no. 02, May 2013 (2013-05-01), US, pages 32 - 38, XP055413322, ISSN: 2160-5815, DOI: 10.4236/wjv.2013.32006
- GALINA V. MUKAMOLOVA ET AL: "A family of autocrine growth factors in Mycobacterium tuberculosis : Autocrine growth factors in Mycobacterium tuberculosis", MOLECULAR MICROBIOLOGY., vol. 46, no. 3, 1 November 2002 (2002-11-01), GB, pages 623 - 635, XP055413302, ISSN: 0950-382X, DOI: 10.1046/j.1365-2958.2002.03184.x
- OBOLBEK TURAPOV ET AL: "The In Vivo Environment Accelerates Generation of Resuscitation-Promoting Factor-Dependent Mycobacteria", AMERICAN JOURNAL OF RESPIRATORY AND CRITICAL CARE MEDICINE., vol. 190, no. 12, 15 December 2014 (2014-12-15), US, pages 1455 - 1457, XP055413399, ISSN: 1073-449X, DOI: 10.1164/rccm.201407-1289LE
- E. KOLWIJCK ET AL: "Early stationary phase culture supernatant accelerates growth of sputum cultures collected after initiation of anti-tuberculosis treatment", CLINICAL MICROBIOLOGY AND INFECTION., vol. 20, no. 7, 1 July 2014 (2014-07-01), United Kingdom, Switzerland, pages O418 - O420, XP055413400, ISSN: 1198-743X, DOI: 10.1111/1469-0691.12441
- SUN ZHONGHE ET AL: "Spent culture supernatant of Mycobacterium tuberculosis H37Ra improves viability of aged cultures of this strain and allows small inocula to initiate growth", JOURNAL OF BACTERIOLOGY, AMERICAN SOCIETY FOR MICROBIOLOGY, US, vol. 181, no. 24, 1 December 1999 (1999-12-01), pages 7626 - 7628, XP002199133, ISSN: 0021-9193

## Description

La présente invention concerne un nouveau milieu de transport et et/ou conservation des mycobactéries, en particulier des mycobactéries du complexe *Mycobacterium tuberculosis,* permettant de préserver la viabilité des mycobactéries et permettant leur pré-incubation pendant le transport du prélèvement et de réduire de façon significative les délais d'isolement par culture et donc le délai de diagnostic des mycobactérioses, en particulier de la tuberculose.

Les mycobactéries sont des bactéries classées dans le phylum des *Actinobacteria* par le séquençage du gène 16S ARNr et par les analyses dites "multilocus phylogeny" [1] et par les analyses génomiques et caractérisées par la présence d'acides mycoliques dans leur paroi, ce qui leur confère une affinité tinctoriale particulière (coloration de Ziehl-Neelsen), et par un chromosome à haut G + C % > 60 % [2]. Le genre bactérien *Mycobacterium* comporte plus de cent soixante espèces, dont des espèces environnementales isolées d'environnements inertes (sol, eau), des espèces associées aux animaux et des espèces majoritairement isolées chez l'homme, dont *Mycobacterium leprae,* agent de la lèpre [3] et les espèces du complexe *Mycobacterium tuberculosis* responsables de la tuberculose. Certaines espèces environnementales sont responsables d'infections opportunistes chez l'homme (les espèces du complexe *Mycobacterium avium* par exemple) et certaines espèces sont responsables de zoonoses, y compris certaines espèces du complexe *Mycobacterium tuberculosis.*

Le diagnostic des mycobactérioses repose sur l'isolement et la culture de l'une des espèces du genre *Mycobacterium* à partir d'un prélèvement clinique réalisé chez l'homme ou chez l'animal. De ce point de vue, le genre *Mycobacterium* comporte :
- une espèce non-cultivée en routine de laboratoire à savoir *Mycobacterium leprae,*
- des espèces de croissance rapide à savoir notamment *Mycobacterium fortuitum,* et *Mycobacterium abscessus,* donnant des colonies visibles en moins de sept jours de culture, et
- des espèces à croissance lente à savoir entre autres les espèces du complexe *Mycobacterium avium, Mycobacterium ulcerans* et les espèces du complexe *Mycobacterium tuberculosis,* donnant des colonies visibles en plus de sept jours de culture, soit en pratique de routine, entre 3 et 8 semaines de culture, notamment avec des milieux de culture Middlebrook.

En médecine humaine, les espèces du complexe *Mycobacterium avium* sont détectables après 10-20 jours de culture et les espèces du complexe *Mycobacterium tuberculosis* requièrent 10-100 organismes viables par ml de prélèvement et une médiane de 2 semaines de culture pour obtenir 100% de prélèvements positifs [4]. Etant donné l'importance numérique et la gravité des cas de tuberculose humaine, c'est sur le diagnostic de la tuberculose que les améliorations des techniques de laboratoire sont particulièrement sensibles. En effet, la tuberculose est une maladie infectieuse de l'homme et des animaux due à l'une des sept espèces du complexe *Mycobacterium tuberculosis* : *Mycobacterium tuberculosis, Mycobacterium bovis* (et les souches BCG qui en sont dérivées), *Mycobacterium africanum, Mycobacterium canettii, Mycobacterium caprae, Mycobacterium microti,* et *Mycobacterium pinnipedii* [5]. L'Organisation Mondiale de la Santé (OMS) a estimé que 9,2 millions de nouveaux cas de tuberculose ont été détectés et que celle-ci a été responsable de 1,6 millions de décès en 2015 [6]. Ces chiffres soulignent l'importance qu'il y a à optimiser le diagnostic microbiologique de la tuberculose afin d'améliorer la prise en charge médicale des patients et de leur entourage.

L'isolement et la culture des mycobactéries du complexe *Mycobacterium tuberculosis* sont réalisées à partir de prélèvements cliniques obtenus chez un patient présentant des signes et des symptômes évocateurs de la tuberculose. La forme clinique la plus fréquente, qui est aussi la seule forme clinique contagieuse, est la tuberculose pulmonaire qui est diagnostiquée par l'isolement et la culture d'une mycobactérie du complexe *Mycobacterium tuberculosis à* partir d'un prélèvement respiratoire tel que l'expectoration, l'aspiration bronchique, le liquide de lavage broncho-alvéolaire obtenu sur bronchoscopie, voire la biopsie pulmonaire. Chez les patients ne produisant pas d'expectoration, les selles peuvent être utilisées comme une alternative pour l'isolement et la culture des mycobactéries du complexe *Mycobacterium tuberculosis* en cas de tuberculose pulmonaire [7]. Il existe d'autres formes cliniques de tuberculose, en particulier la tuberculose ganglionnaire, mais également les tuberculoses osseuses (Mal de Pott) ainsi que les tuberculoses digestives. En fonction de la forme clinique, différents prélèvements cliniques peuvent être adressés au laboratoire pour isoler et cultiver les mycobactéries du complexe *Mycobacterium tuberculosis* et diagnostiquer les formes extrapulmonaires.

Ces prélèvements réalisés chez un patient suspect de présenter une infection à mycobactéries, en particulier une tuberculose due à une des espèces du complexe *Mycobacterium tuberculosis,* sont habituellement recueillis dans des récipients secs, c'est-à-dire ne contenant aucun milieu de transport, dans le but de ne pas diluer l'échantillon clinique; et sont ensuite transportés à température ambiante dans un laboratoire de mycobactériologie dans lequel les différentes opérations de laboratoire visant à déterminer la présence de mycobactéries, leur identification et leur sensibilité aux antibiotiques sont réalisées. Le délai de transport dudit échantillon varie de quelques heures à plusieurs jours, voire plusieurs semaines s'il s'agit d'échantillons manipulés dans un pays différent de celui dans lequel le laboratoire est localisé. Dans ces conditions, il a été mesuré une décroissance très rapide de l'inoculum (la quantité) de mycobactéries viables dans l'échantillon laissé à température ambiante, et par conséquent une diminution très importante du succès de la culture des mycobactéries : la viabilité de *Mycobacterium tuberculosis* à température ambiante décroit de 3 à 4 log due à une croissance de 1 à 1,5 log des contaminants [8].

Pour pallier cette situation, il convient de limiter le plus possible le délai de culture de l'échantillon mais cela est contingent de facteurs extérieurs non-maitrisés. De façon alternative, plusieurs modalités de transport ont été proposées comportant le transport réfrigéré voire congelé du prélèvement : en effet, la viabilité de *Mycobacterium tuberculosis* est entièrement préservée à -20°C [9] et est préservée à 94.4% à 2-4°C [10]. Cependant, la mise en œuvre est difficile en routine singulièrement dans les situations où elle serait le plus utile. La perte de viabilité de *Mycobacterium tuberculosis* étant en partie liée à la croissance de bactéries ou levures contaminantes (tels que bactéries du genre *Streptococcus,* bactéries du genre *Pseudomonas,* levures du genre *Candida*)*,* il a été proposé que pour un délai de transport supérieur à 4 heures, le prélèvement soit placé dans un milieu de transport contenant un décontaminant [11]. Les décontaminants contenant un ammonium quaternaire tel que le chlorure de cetylpyridinium (CPC, 1%) [12-13], le bromide de cetylpyridinium (CPB, 0.6%) [14], et le gluconate de chlorhexidine (CHX, 1% et 0.7%) [15-16] sont utilisés en routine. La culture de *Mycobacterium tuberculosis à* partir d'échantillons décontaminés par un ammonium quaternaire est significativement supérieure à celle à partir de l'échantillon non décontaminé [11,12, 17-19]. La chlorhexidine préserve 1-2 log de plus de mycobactéries que le NALC-NaOH et le NALC-NaOH-Oxa [16, 20] et décontamine les selles [21] et les expectorations [16]. L'incubation pendant 18-24h dans du phosphate trisodique a été également proposée [22-24].

Cependant, tous les agents décontaminants sont toxiques pour les mycobactéries, ayant donc tendance à diminuer l'inoculum de départ contenu dans l'échantillon biologique. Aucune des solutions actuellement proposées n'étant applicables en routine ni ne donnant de résultats satisfaisant, les prélèvements sont pratiquement tous adressés au laboratoire dans des récipients secs, avec un taux de succès de la culture de *Mycobacterium tuberculosis* qui décroit au-delà de 4 heures de délai.

Un premier but de la présente invention est de fournir un milieu de transport et/ou conservation d'échantillons de prélèvements cliniques suspectés de contenir *Mycobacterium tuberculosis,* qui palie les inconvénients des différentes solutions proposées précédemment, à savoir un maintien voire une augmentation du nombre de mycobactéries dans le dit milieu dans le temps notamment pendant plus de 4 heures voire au-delà de 48 h à température ambiante de façon à obtenir un inoculum de départ accru pour la culture subséquente.

On entend ci-après par « milieu de transport », un milieu de transport et/ou de conservation. Un tel milieu de transport se distingue essentiellement d'un milieu de culture en ce que ce dernier présente des facteurs de croissances additionnels tels qu'explicités ci-après. Plus particulièrement, la présente invention concerne un milieu de transport et/ou de conservation à température ambiante qui doit préserver la viabilité des mycobactéries après au moins 48h, de préférence au moins 72h, voire de préférence encore au moins 7 jours dans ledit milieu de transport et conservation pour permettre l'isolement et la détection voire la culture ultérieure des dites mycobactéries.

D'autre part, pour l'isolement et la culture des mycobactéries du complexe *Mycobacterium tuberculosis,* on dispose de milieux solides, de milieux liquides, et de milieux biphasiques comportant une phase liquide et une phase solide [25]. Les milieux solides sont fabriqués à base de gélose ou d'agar. Les milieux contenant de l'œuf entier sont l'utilisation très courante et le milieu le plus utilisé est le milieu de Löwenstein Jensen. Ce milieu, comme les autres milieux contenant de l'œuf, contient du vert malachite qui participe à inhiber la croissance des microorganismes contaminants. Plusieurs formulations contenant des concentrations variables de vert malachite ont été proposées avec comme résultat constant qu'une diminution de la concentration de vert malachite augmente le ratio de contamination du milieu et une augmentation de la concentration de vert malachite tend à diminuer l'isolement et la culture des mycobactéries du groupe tuberculeux. Une deuxième catégorie de milieux solides sont les milieux à l'agar en particulier le milieu Middlebrook 7H10 et le milieu 7H11 (milieu 7H10 plus 0,1% de caséine hydrolysée). Le milieu de Middlebrook contient 2% de glycérol, qui facilite la culture des mycobactéries du complexe *Mycobacterium avium.* Les milieux liquides correspondent essentiellement au milieu Middlebrook 7H9.

Dans le brevet EP 2 364 357, on a décrit une formulation de milieux de culture permettant l'isolement plus rapide des mycobactéries du complexe *Mycobacterium tuberculosis* et des autres mycobactéries, permettant notamment une détection et une identification détectable visuellement à l'œil nu en moins de 15 jours, voire même en 10 jours, à partir d'échantillons de prélèvement cliniques. Cette formulation de EP 2 364 357 permet l'isolement des mycobactéries avec la capacité de détecter visuellement à l'œil nu les mycobactéries en croissance, aussi bien en milieu liquide adapté aux automates de détection qu'en milieu solide pour une détection manuelle, (notamment les compositions des exemples 1B (milieu liquide) et 2B (milieu solide). Ces formulations du fait de la présence de lécithine, sont, de surcroît, compatibles avec une décontamination à la chlorhexidine dans le cas de prélèvements cliniques pouvant comprendre des bactéries de la flore commensale risquant d'inhiber la croissance des mycobactéries [26].

Plus précisément, dans EP 2 364 357, les milieux de culture de mycobactéries, comprennent des facteurs de croissance de mycobactéries et, de préférence, des antibiotiques sans activité à l'égard des mycobactéries, caractérisés en ce qu'ils comprennent les composants additionnels suivants :
- de la lécithine, et
- du sang défibriné, et
- du sérum de veau fœtal décomplémenté.

Cependant, la mise en œuvre de sang, rend ces formulations difficiles à manipuler lors de sa fabrication industrielle et limite le délai de péremption dudit milieu car le sang doit être mis en œuvre dans un délai inférieur à 24h et conservé à 4°C pour rester frais. En outre, le sang doit être prélevé en présence d'anticoagulants pour éviter sa coagulation, ces anticoagulants pouvant avoir un effet négatif sur la croissance des mycobactéries.

Dans WO 2016/142626, on a décrit un milieu solide conservant les caractéristiques et performances des milieux décrit ci-dessus, mais ne comportant pas de sang, dans lequel on a remplacé le sérum de veau fœtal par du sérum d'agneau combiné à un colorant synthétique sans activité contre les mycobactéries.

Cependant, l'isolement des mycobactéries du complexe *Mycobacterium tuberculosis* reste lent puisque la totalité des souches des différentes espèces du complexe *Mycobacterium tuberculosis* est isolée dans un délai médian de 1 à 2 semaines [25]. Tout gain de temps sur ce délai représente donc toujours une amélioration significative du diagnostic de laboratoire de la tuberculose et les autres infections à mycobactérie.

WO 02/45736 décrit un surnageant (nommé ESPSN) collecté dans la phase précoce stationnaire d'une culture de mycobactéries du complexe Mycobacterium tuberculosis. L'ESPSN est utilisé pour pour raviver les bacilles Mycobacterium dormants et pour permettre l'utilisation d'inoculums plus petits.

Un autre but de la présente invention est de fournir de nouveaux procédés et milieu de culture de mycobactéries conservant les caractéristiques et performances des milieux de culture décrits ci-dessus en termes de gain de temps de culture, mais ne comportant pas de sang qui est un produit biologique non-standardisable et dont la production régulière est rendue difficile par la nature même de l'échantillon et les contraintes réglementaires qui s'y attachent.

Après de nombreuses tentatives, il a été mis au point selon la présente invention, une nouvelle formulation de milieux de transport et conservation conforme aux buts de la présente invention, à savoir :
- un milieu de transport et conservation permettant d'augmenter la viabilité des mycobactéries du complexe *Mycobacterium tuberculosis,* et
- un milieu de culture permettant la culture plus rapide des mycobactéries du complexe *Mycobacterium tuberculosis,* notamment permettant un gain de temps de culture pouvant aller jusqu'à deux-tiers du délai standard après transport ou conservation dans le dit milieu de transport.

Plus précisément, la présente invention fournit un milieu de transport et/ou conservation contenant ou destiné à contenir un échantillon de mycobactéries du complexe *Mycobacterium tuberculosis,* notamment la bactérie *Mycobacterium tuberculosis,* pour le transport et/ou la conservation de dites mycobactéries, comprenant un milieu de culture de base de mycobactéries comprenant des composés antibiotiques sans activité à l'égard des mycobactéries, et au moins un agent décontaminant caractérisé en ce qu'il comprend du surnageant complet de culture de *Mycobacterium bovis* souche BCG comme composant additionnel, dans une proportion d'au moins 2,5% en volume, ledit surnageant étant récolté avant la phase stationnaire de croissance après pas plus de deux semaines de culture.

*Mycobacterium bovis* est la bactérie responsable d'une maladie dite « tuberculose bovine » (TB) qui affecte les bovins d'élevage et sauvages, mais aussi de nombreux animaux sauvages mammifères autres que les bovins. C'est l'une des formes du bacille de Koch (BK) responsables de diverses formes de tuberculose humaine. Elle en est très proche génétiquement, mais avec un agencement différent des gènes. C'est à partir d'une forme atténuée de ce bacille qu'est produit le vaccin BCG. Le vaccin bilié de Calmette et Guérin, le plus souvent dénommé vaccin « BCG » est un vaccin contre la tuberculose. Il est préparé à partir d'une souche atténuée de bacille tuberculeux bovin (*Mycobacterium bovis*) vivant qui a perdu sa virulence sur l'homme par culture spéciale sur des milieux artificiels pendant des années. Ce bacille proche de *Mycobacterium tuberculosis,* responsable de la tuberculose humaine, confère une antigénicité croisée suffisamment forte pour devenir un vaccin effectif pour la prévention de la tuberculose humaine. Il a également été utilisé en médecine vétérinaire.

Plusieurs souches *Mycobacterium bovis* BCG sont disponibles dans le commerce, notamment la souche de *Mycobacterium bovis* BCG Pasteur 1173P2 disponible notamment à l'ATCC sous le n°35734.

Selon la présente invention, on a donc découvert que l'ajout du surnageant complet de culture de *Mycobacterium bovis* souche BCG répondait aux buts de la présente invention, plus particulièrement dans le cas d'un milieu liquide comme il sera explicité ci-après.

La formulation de milieu de transport et conservation de mycobactéries du complexe *Mycobacterium tuberculosis,* notamment sous forme d'échantillons de prélèvements cliniques d'expectoration en contenant selon l'invention permet d'accroitre la viabilité et constitue en outre un milieu de pré-incubation favorisant une croissance accrue dans une culture subséquente l'isolement des mycobactéries.

La formulation de milieu de transport selon l'invention permet l'isolement et la culture ultérieure des mycobactéries, aussi bien en milieu liquide adapté aux automates de détection qu'en milieu solide pour une détection manuelle ou automatisée notamment par l'utilisation de scanner de détection [26,27].

Le surnageant de culture de *Mycobacterium bovis* souche BCG n'est pas un réactif habituellement utilisé pour la culture bactérienne. Il est avantageusement obtenu à partir de la culture d'une mycobactérie *Mycobacterium bovis* laquelle peut-être réglementairement cultivée dans un laboratoire de classe de sécurité 1 ou 2, étant par ailleurs un vaccin largement utilisé chez l'homme dans deux indications, la prévention de la tuberculose et l'immunothérapie du cancer de la vessie.

On entend ici par « surnageant complet », la totalité des produits extracellulaires sécrétés obtenus par filtration stérilisante d'une culture de la dite bactérie sans autre fractionnement que ladite filtration éliminant les bactéries entières notamment avec un filtre de 0.22µm.

On comprend que le milieu transport de mycobactéries selon l'invention est constitué d'un milieu de culture de base, connu pour la culture des mycobactéries, comprenant notamment des sels minéraux, sucres, acides aminés, protéines et vitamines.

Ces composants sont, notamment, les composants contenus dans les milieux de culture de mycobactéries dénommés milieux Middlebrook.

En pratique, ce milieu de transport se présente initialement sous forme d'un lyophilisat desdits composants listés ci-dessus, destiné à être dilué dans de l'eau distillée pour former le milieu de culture selon l'invention.

Selon la présente invention, avantageusement le surnageant complet de culture de *Mycobacterium bovis* souche BCG est récolté avant la phase stationnaire de croissance après pas plus de 2 semaines de culture, de préférence pas plus de 10 jours de culture. Selon la présente invention, on peut utiliser le surnageant complet du fait que celui-ci est prélevé de façon très précoce à pas plus de 10 jours de culture avant la phase stationnaire de la croissance de la bactérie au cours de laquelle la composition du surnageant est modifiée.

Plus particulièrement, le dit surnageant complet de culture de *Mycobacterium bovis* est récolté à partir d'une culture de *Mycobacterium bovis* souche BCG dans un premier milieu de culture, de préférence après pas plus 10 jours de culture, ledit premier milieu de culture présentant les mêmes composants que ledit milieu de culture de base de mycobactérie dans lequel est ajouté le dit surnageant.

Plus particulièrement encore, le dit surnageant complet de culture est récolté à partir d'une culture de *Mycobacterium bovis* souche BCG dans un milieu liquide selon le protocole de culture suivant :
1) une suspension de *Mycobacterium bovis* souche BCG, de préférence calibrée à 10⁶ bactéries/mL par densité optique, est préparée à partir d'une colonie isolée sur un milieu de culture solide, notamment un milieu de culture gélosé contenant de l'œuf, de la fécule de pomme de terre et du glycérol tel que le milieu Coletsos,
2) une suspension bactérienne est inoculée dans un milieu de culture liquide, notamment Middlebrook 7H9 dans un flacon ; puis
3) le flacon contenant la suspension ainsi inoculée a été incubé dans un automate de culture qui détecte automatiquement la croissance des mycobactéries sur la base de la consommation en oxygène à l'intérieur du flacon, et
4) lorsque la croissance a été détectée (notamment après 7 à 10 jours de culture), l'identification de la souche est confirmée pour vérifier la présence de mycobactéries et l'absence de contaminants notamment par une coloration de Gram pour vérifier l'absence de contaminants et par une coloration de Ziehl-Neelsen pour vérifier la présence de mycobactéries, et
5) Le surnageant de culture est filtré à l'aide d'un filtre 0.22 µm afin d'éliminer les mycobactéries, de préférence un filtre 0.22 µm, et le filtrat est conservé de préférence à -80°C.

Plus particulièrement encore, le dit surnageant de culture est récolté à partir d'une culture de *Mycobacterium bovis* souche BCG dans un milieu liquide Middlebrook 7H9 selon le protocole de culture décrit à l'exemple 1 ci-après.

Comme mentionné ci-dessus, selon une autre caractéristique avantageuse et originale de la présente invention, de façon surprenante, la dispersion des mycobactéries par dilution de l'échantillon de prélèvement clinique dans lequel des mycobactéries du complexe *Mycobacterium tuberculosis* se trouvent sous forme d'agrégats de mycobactéries comme cela est le cas dans les prélèvements cliniques d'expectoration favorise la viabilité des mycobactéries dans un dit milieu de transport liquide en combinaison avec un surnageant de *Mycobacterium bovis* BCG, en opposition à ce qui est généralement admis s'agissant de la dilution des échantillons bactériens pour les milieux de transport. Et de surcroit, un tel milieu de transport selon l'invention constitue un milieu de pré-incubation qui favorise la croissance ultérieure dans un milieu de culture selon l'invention.

De préférence, un milieu de transport et/ou culture de mycobactéries selon l'invention est un milieu liquide comprenant en outre au moins un agent dispersant apte à disperser les agrégats de mycobactéries d'un échantillon clinique de mycobactéries, de préférence ledit agent dispersant étant un détergent tel que du Tween 80^{™} (à base de polyéthylène sorbitol ester) à une concentration d'au moins 0,01% et à concentration inférieure à 1% dans le dit milieu de transport, de préférence encore inférieure à 0.1%.

Plus particulièrement, un dit milieu liquide de transport de mycobactéries selon l'invention comprend en outre :
- au moins un agent décontaminant choisi parmi la Chlorhexidine et le vert de malachite, et
- la lécithine d'œuf, et
- au moins une source de carbone, de préférence du glycérol, et
- des composés antibiotiques, de préférence la colimycine et la pénicilline G, et
- au moins un composé antifongique, de préférence l'amphotéricine B.

Le gycérol constitue une source de carbone additionnelle et assure ainsi les besoins nutritifs nécessaires à la culture des mycobactéries.

La lécithine d'œuf protège les mycobactéries de l'agent décontaminant tel que Chlorhexidine.

Plus particulièrement encore, le dit milieu selon l'invention comprend un milieu de culture de mycobactéries de base comprenant, outre de l'eau distillée, les composants suivants : sulfate d'ammonium, sulfate de magnésium, sulfate de cuivre, sulfate de zinc, citrate de sodium, citrate d'ammonium ferrique, chlorure de sodium, chlorure de calcium, phosphate monopotassique, phosphate disodique, acide L-glutamique, biotine et pyridoxine.

Plus particulièrement encore, le dit milieu selon l'invention comprend lesdits antibiotiques sans activité antimycobactérienne aux concentrations employées, de préférence la colimycine et la pénicilline G et un antifongique, de préférence l'amphotéricine B.

Plus particulièrement encore, le dit milieu de transport et/ou culture de mycobactéries selon l'invention comprend un milieu de culture de base de mycobactérie qui est un milieu liquide Middlebrook de référence 7H9.

Plus particulièrement, un milieu liquide de transport de mycobactéries selon l'invention comprend les composants suivants, de préférence dans les quantités et proportions pondérales ou volumiques suivantes pour 1L:

| | |
|---|---|
| - Middlebrook 7H9 | : 4,7 g (0.47%) |
| - Glycérol | : 4 mL (0,4 %) |
| - Lécithine d'œuf | : 5 g (0.5%) |
| - OADC | : 100 mL (10%) |
| - Chlorhexidine solution 20% | : 15 mL (1.5%) |
| - Tween 80 | : 0.5 mL (0.05%) |
| - Vert malachite | : 0.25 g (0.025%) |
| - Colimycine | : 0.05 g (0.005%) |
| - Pénicilline G | : 2.5 g (0.25%) |
| - Amphotéricine B | : 0.01 g (0.001%) |
| - Surnageant de culture de M. bovis BCG | : 25 mL (2,5%) |

On entend ici par OADC, la combinaison de facteur de viabilité des mycobactéries : acide oléique, albumine bovine, dextrose et catalase.

On entend ici par milieu Middlebrook 7H9, le milieu constitué pour 1000 mL de milieu :
Eau distillée stérile : 750 ml
Sulfate d'ammonium : 0,5 g
Acide L-glutamique : 0,5 g
Citrate de sodium : 0,1 g
Pyridoxine : 1 mg
Biotine : 0,5 mg
Phosphate disodique : 2,5 g
Phosphate monopotassique : 1 g
Citrate d'ammonium ferrique : 0,04 g
Sulfate de magnésium : 0,05 g
Chlorure de calcium : 0,5 mg
Sulfate de zinc : 1 mg
Sulfate de cuivre : 1 mg
Glycérol: 4 mL
Polysorbate 80 : 1 g

Dans un autre mode de réalisation particulier, un milieu destiné à la culture ultérieure de dites mycobactéries comprend les mêmes composants que le dit milieu de transport et en outre des facteurs de croissance de mycobactéries additionnels suivants : hydrolysat de caséine, protéose peptone, extrait de levure, extrait de bœuf, amidon de pommes de terre, glucose, sérum d'agneau inactivé, huile d'olive et au moins un composé antioxydant de préférence choisi parmi L-cystéine, acide urique, acide ascorbique et L-glutathion.

Plus particulièrement encore, ledit milieu de culture est un milieu de culture solide contenant un produit gélifiant choisi de préférence parmi les géloses et agar, de préférence dans une proportion pondérale de 0.5 à 5%, de préférence encore de 1 à 2%.

Dans une variante, ledit milieu de culture comprend un milieu de culture de base solide de type Middlebrook de référence 7H10 comprenant les composant du milieu Middlebrook de référence 7H9 additionné de dit produit gélifiant.

Plus particulièrement encore, ledit milieu de culture de mycobactéries selon l'invention comprend des facteurs de croissance de mycobactéries additionnels suivants : acide oléique, albumine bovine, de préférence la fraction V de l'albumine bovine, dextrose, catalase.

Plus particulièrement encore, le dit milieu de culture de mycobactéries ne comprend ni composés antibiotiques ni composés antifongiques. Ce mode de réalisation est avantageux lorsque l'échantillon de dites mycobactéries a été transporté et/ou conservé dans un milieu de transport selon l'invention contenant desdits composés antibiotiques et composés antifongiques.

La présente invention fournit également un procédé de transport et/ou conservation de mycobactérie, de préférence la bactérie *Mycobacterium tuberculosis,* dans un dit échantillon de prélèvement clinique en contenant assurant la viabilité de mycobactérie pendant au moins 4 heures de préférence au moins 48 heures, caractérisé en ce que l'on réalise le transport et/ou conservation dans un dit milieu de transport et/ou conservation selon l'invention contenant un dit surnageant de culture obtenu à partir d'une culture de *Mycobacterium bovis* souche BCG de préférence dans un premier milieu de culture présentant les mêmes composants que ledit milieu de culture de base de mycobactérie.

La présente invention permet de mettre en œuvre un procédé de culture d'une mycobactérie à l'aide d'un milieu de culture de mycobactéries selon l'invention dans lequel on chauffe un échantillon contenant desdites mycobactérie, dans un dit milieu de culture de mycobactéries, à une température de 30 à 37°C, appropriée pour la culture de l'espèce de mycobactérie contenue dans l'échantillon.

Plus particulièrement, on cultive un échantillon contenant une bactérie du complexe *Mycobacterium tuberculosis à* une température de 37°C dans un dit milieu de culture de mycobactéries.

Plus particulièrement encore, on réalise les étapes suivantes:
- on effectue la culture d'un échantillon de prélèvement biologique pouvant contenir des mycobactéries jusqu'à ce qu'une croissance de bactéries soit détectable, et
- on identifie que la bactérie détectée est une bactérie du genre mycobactérie par un test de coloration, et
- le cas échéant, on identifie l'espèce de ladite mycobactérie par des moyens d'analyse moléculaire, de préférence par spectrométrie de masse.

Un milieu de transport de dites mycobactéries selon l'invention permet de détecter ultérieurement après culture une croissance de bactérie du complexe *Mycobacterium tuberculosis* en moins de 15 jours, de préférence en pas plus de 10 jours, permettant la détection de 50% des mycobactéries en moins d'une semaine.

Plus particulièrement encore, ledit milieu de culture de mycobactéries est un milieu de culture liquide de mycobactéries et en ce qu'à partir d'un inoculât de 10⁶/mL mycobactéries, on détecte une croissance de bactérie du complexe *Mycobacterium tuberculosis* en pas plus de 7 jours.

La présente invention fournit également un procédé de transport et de culture d'un échantillon de prélèvement clinique contenant ou susceptible de contenir des mycobactéries du complexe *Mycobacterium tuberculosis* de préférence la bactérie *Mycobacterium tuberculosis,* dans lequel :
a) on réalise le transport et/ou la conservation dudit échantillon de prélèvement clinique dilué dans un milieu liquide de transport et conservation selon l'invention contenant des composés antibiotiques et au moins un composé antifongique, et
b) on réalise ensuite la culture des mycobactéries du dit échantillon de prélèvement clinique dilué dans un milieu de culture de dites mycobactéries, de préférence un milieu de culture solide ne contenant ni composés antibiotiques ni composé antifongique.

Plus particulièrement, à l'étape b), ledit milieu de culture dites mycobactéries comprend les mêmes composants que le dit milieu de transport à l'exception des dits agents décontaminants et en outre des facteurs de croissance de mycobactéries additionnels suivants : hydrolysat de caséine, protéose peptone, extrait de levure, extrait de bœuf, amidon de pommes de terre, glucose, sérum d'agneau inactivé, huile d'olive et au moins un composé antioxydant de préférence choisi parmi L-cystéine, acide urique, acide ascorbique et L-glutathion.

D'autre caractéristiques et avantages de la présente invention apparaitront à la lumière des exemples illustratifs ci-après.

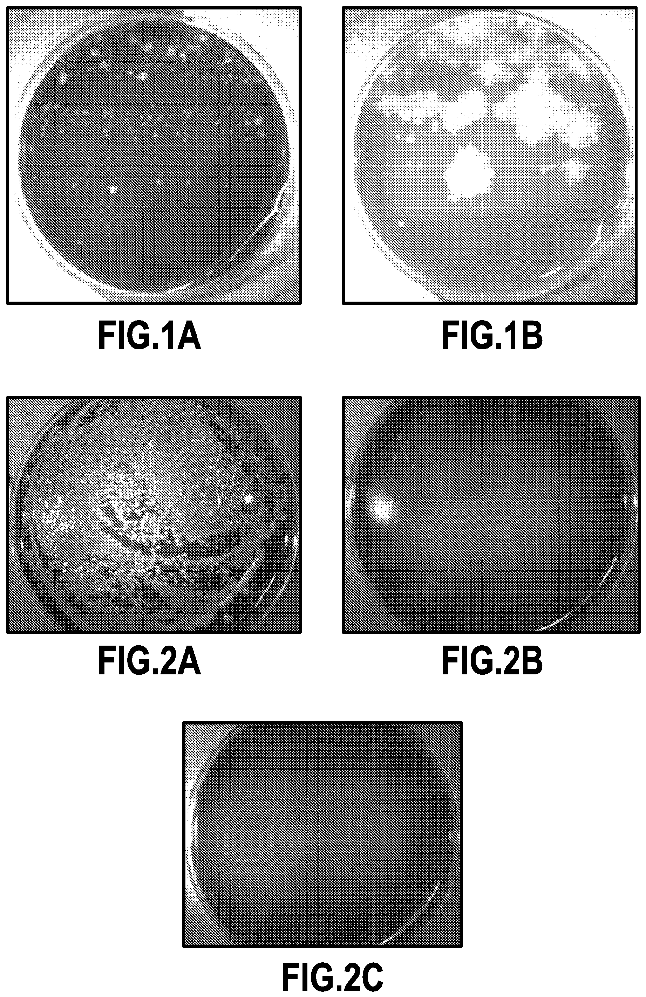

A l'exemple 1, Les figures 1A et 1B montrent les résultats de croissance des mycobactéries avec un milieu gélose au sang (Figure 1A) et un milieu contenant du sérum d'agneau décomplémenté, des antioxydants et du surnageant de BCG (dénommé milieu COSMO) (Figure 1B), après 16 jours d'incubation.

A l'exemple 2, les figures 2A à 2C montrent l'impact du milieu de transport sur la décontamination après plusieurs heures d'incubation (figure 2B : après 1 heure d'incubation dans le milieu de transport selon l'invention, figure 2C : après 10 heure d'incubation dans le milieu de transport selon l'invention) en comparaison à l'absence de milieu de transport (figure 2A).

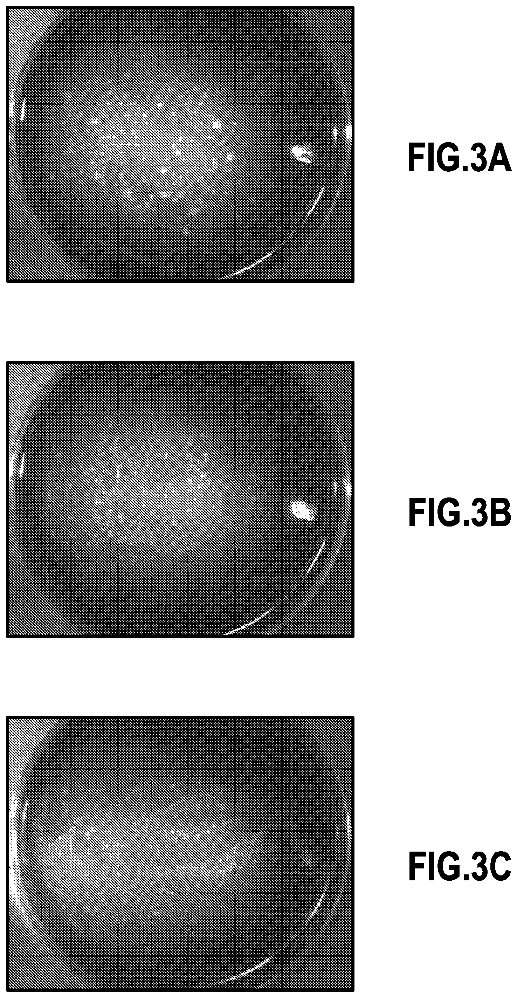

A l'exemple 2, les figures 3A à 3C montrent l'impact du milieu de transport sur la survie de *Mycobacterium tuberculosis* en comparaison à un milieu PBS, le nombre de colonies étant plus important lorsque le prélèvement a été pré-incubé dans le milieu de transport dès 2 heures d'incubation (figure 3B) et après 10h d'incubation (figure 3C), par comparaison à un milieu PBS comme témoin négatif (figure 3A).

### EXEMPLE 1 : Milieu de culture de Mycobactéries.

Les inventeurs ont étudié la culture de trois souches de *Mycobacterium tuberculosis* en milieu liquide, complémenté ou non par du surnageant de *Mycobacterium bovis* souche BCG.

### 1) Préparation du surnageant de culture de Mycobacterium bovis souche BCG

Une souche BCG Pasteur de *Mycobacterium bovis* 1173P2 dont l'identification a été confirmée par analyse de son profil peptidique obtenu par spectrométrie de masse MALDI-TOF [Zingue D, Flaudrops C, Drancourt M. Direct matrix-assisted laser desorption ionisation time-of-flight mass spectrometry identification of mycobacteria from colonies. Eur J Clin Microbiol Infect Dis. 2016 Dec;35(12):1983-1987] a été inoculée à la concentration de 10⁶ mycobactéries/mL dans un milieu liquide Middelbrook 7H9 décrit précédemment (flacons MGIT, Becton Dickinson, Le-Pont-de-Claix, France).

A partir d'une colonie isolée sur un milieu Coletsos, une suspension de BCG calibrée à 10⁶ bactéries/mL par densité optique a été préparée. 20 µL de cette suspension bactérienne ont été inoculés dans un milieu liquide Middlebrook 7H9 (flacons MGIT, Becton Dickinson, Le-Pont-de-Claix, France). Le flacon ainsi inoculé a été incubé dans un automate de culture BACTEC MGIT 960 (Becton Dickinson, Le-Pont-de-Claix, France) qui détecte automatiquement la croissance des mycobactéries sur la base de la consommation en oxygène à l'intérieur du flacon. Lorsque la croissance a été détectée (après 7 à 10 jours de culture), l'identification de la souche a été confirmée par une coloration de Gram qui a montré l'absence de contaminants et par une coloration de Ziehl-Neelsen qui a montré la présence de mycobactéries. Le surnageant de culture a été filtré à l'aide d'un filtre 0.22 µm afin d'éliminer les mycobactéries et le filtrat aliquoté a été conservé à -80°C.

### 2) Culture de Mycobacterium tuberculosis

Les inventeurs ont réalisé des suspensions mycobactériennes de cinq souches identifiées de *Mycobacterium tuberculosis* dont l'identification a été confirmée par analyse de son profil peptidique obtenu par spectrométrie de masse MALDI-TOF [Zingue D, Flaudrops C, Drancourt M. Direct matrix-assisted laser desorption ionisation time-of-flight mass spectrometry identification of mycobacteria from colonies. Eur J Clin Microbiol Infect Dis. 2016 Dec;35(12):1983-1987] et déposée dans la collection des souches de mycobactéries de l'URMITE de la faculté de médecine La Timone, Marseille, comportant la souche de référence *Mycobacterium tuberculosis* H37Rv et de 4 souches cliniques isolées à partir d'expectorations collectées chez quatre patients différents présentant une tuberculose pulmonaire. Ces suspensions ont été calibrées par densité optique de 10⁶ mycobactéries/mL à 10² mycobactéries/mL.

Ensuite, 500 µL de chaque suspension mycobactérienne ont été inoculés dans un flacon pour automate BACTEC MGIT 960 (Becton Dickinson), dans lesquels les inventeurs ont ensuite inoculé 800 µL de bouillon Middlebrook 7H9 (contrôle positif de croissance) ou bien 800 µL de surnageant de culture de *Mycobacterium bovis* BCG, contenant du milieu Middlebrook 7H9. Des flacons inoculés avec uniquement 800 µL de surnageant de culture de *Mycobacterium bovis* BCG, contenant du milieu Middlebrook 7H9, ont été utilisés comme témoins négatifs de croissance. Le volume de 800 µL correspond au dixième du volume final du flacon. Les flacons ont été ensuite incubés dans l'automate de culture BACTEC MGIT 960 pendant 1 mois. Cette expérience a été réalisée trois fois.

Le délai de positivité de la croissance détecté par l'automate BACTEC a été utilisé comme critère de jugement et les flacons détectés positifs ont été confirmés comme des vrais positifs par coloration de Gram éliminant dans tous les cas un contaminant et par coloration de Ziehl retrouvant dans tous les cas la présence de mycobactéries.

Les résultats sont présentés dans le tableau ci-après.

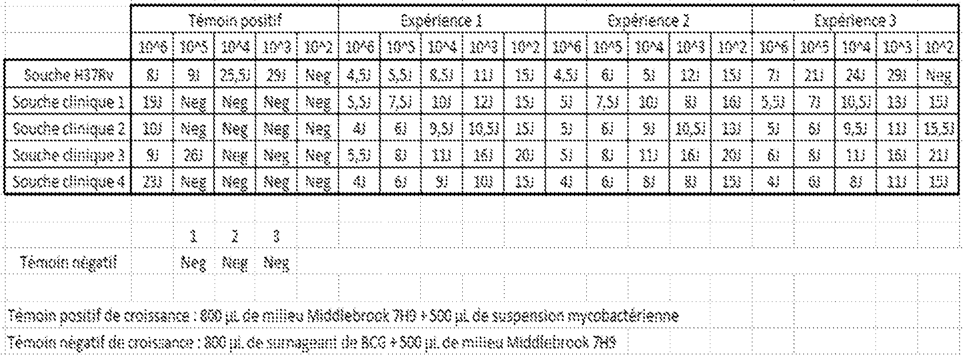

Les inventeurs ont observé que les dilutions calibrées à 10⁶ mycobactéries/mL et incubées en présence de surnageant de BCG étaient détectées positives en moyenne 2 jours (2J) plus tôt pour la souche H37Rv et 3,3 jours (3.3J) plus tôt pour les souches cliniques, respectivement.

Par ailleurs, les dilutions calibrées à 10⁴, 10³ et 10² mycobactéries/mL n'ont pu être détectées positives pour les souches cliniques qu'en présence de surnageant de BCG par l'automate de culture BACTEC MGIT 960.

Les inventeurs ont alors ainsi découvert l'intérêt crucial du surnageant de culture de BCG dans lesdits milieux de transport et de culture compte tenu de son effet dans le délai de positivité de la croissance.

### 3) Comparaison entre un milieu gélose au sang et un milieu supplémenté en sérum d'agneau décomplémenté + antioxydants + surnageant de BCG (milieu COSMO)

### Milieu de culture (milieu COSMO):

Composition finale du milieu liquide (pour 1000 mL)
Eau distillée stérile : 746 ml
Lécithine d'œuf : 5 g
Hydrolysat de caséine : 12 g
Protéose peptone : 5 g
Extrait de levure : 3 g
Extrait de bœuf : 3 g
Amidon de pommes de terre : 1 g
Glucose : 5 g
Chlorure de sodium : 0,1 g
Agar : 13,5 g
Colorant alimentaire synthétique : 4 mL

### 2- Facteurs de croissance additionnels :

Huile d'olive : 250 µL
Sérum d'agneau décomplémenté : 150 mL
Antioxydants :
   - acide urique : 1 g
   - L-cystéine : 0.5 g
   - acide ascorbique : 0.2 g

   - L-glutathion : 0.1 g

### 4- Eléments additionnels de l'invention :

Surnageant de culture de *Mycobacterium bovis* BCG : 100 mL

### Milieu de Columbia agar (Milieu comparatif) :

Eau distillée stérile: 950 mL
Agar : 15 g
Chlorure de sodium : 5 g
Substrat nutritif : 23 g
Amidon : 1 g
Sang de mouton défibriné : 50 mL

Les inventeurs ont réalisé des suspensions mycobactériennes de 5 souches identifiées de *Mycobacterium tuberculosis* dont l'identification a été confirmée par analyse de son profil peptidique obtenu par spectrométrie de masse MALDI-TOF [Zingue D, Flaudrops C, Drancourt M. Direct matrix-assisted laser desorption ionisation time-of-flight mass spectrometry identification of mycobacteria from colonies. Eur J Clin Microbiol Infect Dis. 2016 Dec;35(12):1983-1987] et déposée dans la collection des souches de mycobactéries de l'URMITE, Marseille, comportant 5 souches cliniques isolées à partir d'expectorations collectées chez quatre patients différents présentant une tuberculose pulmonaire. Ces suspensions ont été calibrées par densité optique de 10⁶ mycobactéries/mL à 10² mycobactéries/mL. 10 µL de chaque dilution ont ensuite été déposés sous forme de strie sur un même milieu à l'aide d'une oëse. Les géloses ont été incubées à 37°C sous 5% CO₂ et observées à l'œil tous les jours.

Les inventeurs ont observé que le délai de croissance des mycobactéries était similaire entre le milieu gélose au sang et le milieu contenant du sérum d'agneau décomplémenté, des antioxydants et du surnageant de BCG (milieu COSMO), avec une détection inférieure à 7 jours. Si le délai de croissance est comparable entre ces deux milieux, les inventeurs ont cependant observé que la taille des colonies était beaucoup plus importante sur le milieu COSMO ( figure 1B) que sur le milieu gélose au sang (figure 1A), ce qui permet de réaliser les étapes suivantes de biologie moléculaire nécessaire à l'identification des mycobactéries plus rapidement, le milieu COSMO permettant d'obtenir une importante quantité de matériel génétique en moins de jours. Les figures 1A et 1B montrent les résultats après 16 jours d'incubation avec le milieu gélose au sang (Figure 1A) et le milieu COSMO (Figure 1B).

### EXEMPLE 2 : Milieu de transport et de pré-incubation

1) Les inventeurs ont analysé le délai de croissance de *Mycobacterium tuberculosis à* partir de 10 prélèvements respiratoires collectés chez 10 patients différents présentant une tuberculose pulmonaire, après culture dans un milieu liquide à base Middlebrook 7H9 en parallèle d'un milieu de culture solide gélose au sang. Pour ce faire, 2 mL de crachats et 2 mL de soude (v/v) ont été mis sous agitation pendant 15 minutes dans un tube Falcon de 50 mL. Après agitation, le prélèvement a été neutralisé par l'ajout de tampon à hauteur de 50 mL. Le tube a ensuite été centrifugé 5 minutes à 4000 tours et le surnageant a été jeté de façon à ne conserver que 5 mL de suspension.

500 µL de cette suspension ont été inoculés dans un tube MGIT contenant 800 µL de PANTA et 1 mL de vancomycine (2,5 g/L) puis incubés dans un .automate de culture BD BACTEC^{™} pendant 1 mois. En parallèle, 500 µL de cette même suspension ont été ensemencés au râteau sur COS (gélose Columbia au sang de mouton) et incubés dans un scanner (Advencis, bioMérieux, La Balme les Grottes, France pendant 1 mois également.

On entend ici par PANTA le mélange composé de polymyxine B, d'acide nalidixique, de triméthoprime, d'azlocilline. et d'amphotéricine B (BBL^{™} MGIT^{™} PANTA^{™} Antibiotic Mixture, Becton Dickinson, Le-Pont-de-Claix, France).

Pour limiter le risque de contamination, le milieu COS ne contenant pas d'antibiotiques au contraire du milieu MGIT, 800 µL de PANTA ont ensuite été ajoutés au 5 mL de suspension restants après centrifugation. De la même manière que précédemment, 500 µL de cette même suspension ont été ensemencés au râteau sur COS et incubés dans un scanner pendant 1 mois. Les résultats sont présentés dans le tableau ci-après.

| **SCANNER milieux COS** (prélèvement soudé) | BACTEC MGIT 960 **MGIT** | **SCANNER milieux COS** (prélèvement soudé + PANTA) |
|---|---|---|
| Détection positif | Détection positif | Détection positif |
| semi non détecté | 4j+18h ( Ziehl + ) | 568 ufc (tapis) 12j |
| semi non détecté | 3j+16h ( Ziehl+ ) | 209 ufc (semi) 12j |
| 321 UFC 4,8j 1ère detection : 77H | 4j (Ziehl + ) | neg le 07/10 |
| contamination levure | 4j+23h ( ziehl +) | 600 ufc (tapis) 3,5j |
| stérile au bout de 15j d'incubation | 13j ( Ziehl + ) | neg le 07/10 en 30j |
| stérile au bout de 15j d'incubation | 7j ( Ziehl + ) | neg le 07/10 en 30j |
| | 13j ( Ziehl + ) | neg le 07/10 en 30j |
| | 16+7h ( ziehl+) | neg le 07/10 en 30j |
| | 18+1h ( ziehl + ) | neg le 07/10 en 30j |
| stérile au bout de 30 j | positif en 22 jours | |

Sur les 10 prélèvements testés, 10 ont été détectés par le BACTEC^{™} MGIT 960, alors que seulement 1 l'a été par le scanner lorsque le prélèvement a été uniquement traité à la soude. Cependant, il est à noter que 2 prélèvements supplémentaires ont été détectés par le scanner lorsque ceux-ci ont été traités à la soude en présence de PANTA, mettant en avant le rôle avantageux de la décontamination des échantillons avant leur ensemencement.

Les inventeurs ont observé que le délai de culture était généralement plus court en milieu liquide (Bactec^{™} dans cet exemple) qu'en milieu solide (gélose au sang dans cet exemple). Les inventeurs en ont inféré que de façon surprenante, la dilution du prélèvement en milieu liquide était favorable à la croissance de *Mycobacterium tuberculosis à* partir des prélèvements respiratoires. Ils ont alors compris l'intérêt d'un milieu de transport liquide selon l'invention qui diluerait les prélèvements et ses inhibiteurs à l'aide d'un agent dispersant tel qu'un détergent, comme décrit ci-après, et les décontaminerait en parallèle.

2) Ce milieu de transport et de pré-incubation contient les ingrédients suivants aux quantités et teneurs pondérales ou volumiques suivantes pour 1 litre de milieu :

| | |
|---|---|
| - Middlebrook 7H9 | : 4,7g (0.47%) |
| - Glycérol | : 4 mL (0,4 %) |
| - Lécithine d'œuf | : 5g (0.5%) |
| - OADC | : 100mL (10%) |
| - Chlorhexidine solution 20% | : 15mL (1.5%) |
| - Tween 80 | : 0.5mL (0.05%) |
| - Vert malachite | : 0.25g (0.025%) |
| - Colimycine | : 0.05g (0.005%) |
| - Pénicilline G | : 2.5g (0.25%) |
| - Amphotéricine B | : 0.01g (0.001%) |
| - Surnageant de culture de M. bovis BCG | : 100 mL (10%) |

Les ingrédients sont les suivants :
- Lécithine d'œuf (L-α-phosphatidylcholine) de Sigma (France), reference: P5394-25G
- Middlebrook 7H9 de Sigma (France), référence: M0178-500G
- Glycérol de Sigma (France), référence: G2025-500ML
- OADC de Becton Dickinson (France), référence :BBL Middlebrook 211886
- Chlorhexidine digluconate solution 20% de Sigma (France), référence :C9394-25ML
- Tween 80 de Sigma (France), référence : P8074-500ML
- Vert malachite oxalate Certistain^{®} de Merck (France) référence : PC236355

Ce milieu de transport et de pré-incubation est préparé de la façon suivante pour préparer 1 litre de milieu :
- Emulsifier 5 g de lécithine à 56°C sous agitation avec 780,5 mL d'eau distillée ajoutée de façon progressive.
- Ajouter sous agitation magnétique à 56°C :
   Middlebrook 7H9 : 4,7 g
   Glycérol : 4 mL
   Tween 80^{®} : 0,5 mL
   Vert malachite : 0,250 g
- Autoclaver pendant 15 min à 125°C. Après autoclavage, laisser refroidir.
- Préparer la solution d'antibiotiques en ajoutant :
   15 mL de chlorhexidine à 20%
   Colimycine : 0,05 g
   Pénicilline G : 0,05 g
   Amphotéricine B : 0,01 g
   Surnageant de BCG : 100 mL
   OADC : 100 mL
- Filtrer la solution d'antibiotiques à 0.2 µm et l'ajouter au milieu précédemment autoclavé.
- Répartir 5 mL de milieu par tube.
- Conserver le milieu à 4°C, à l'obscurité.

3) Les inventeurs ont analysé l'impact du milieu de transport sur la décontamination du prélèvement d'une part (paragraphe 3.1) et sur la culture de *Mycobacterium tuberculosis* d'autre part (paragraphe 3.2), après incubation dans le milieu de transport. Pour ce faire, 2 mL d'expectoration Ziehl négative ont été artificiellement infectés avec 200 µL d'une suspension bactérienne de mycobactéries calibrée à 10⁶ par densité optique. Ce prélèvement a ensuite été ajouté à 5 mL de milieu de transport et incubé à 37°C pendant 10 heures. Toutes les heures, 100 µL de suspension ont été prélevés et ensemencés au râteau sur milieu COS. Le témoin négatif, qui n'a pas subi d'étape de pré-incubation, a été réalisé avec 200 µL de suspension mycobactérienne calibrée par densité optique à 10⁶ mycobactéries/mL dilués dans 7 mL de PBS (volume correspondant au volume de milieu de transport + expectoration). 100 µL de suspension ont été prélevés et ensemencés au râteau sur milieu COS.
Les géloses ont été incubées dans une étuve à 37°C sous 5% CO₂.

3.1) Sur les figures 2A à 2C, on montre l'impact du milieu de transport sur la décontamination après plusieurs heures d'incubation en comparaison à l'absence de milieu de transport.

En l'absence de milieu de transport (figure 2A), on observe un tapis bactérien très dense, indiquant que le prélèvement est fortement contaminé par des bacteries autres que mycobatéries et particulièrement les bactéries du genre *Pseudomonas* et les bactéries du genre *Enterococcus.* En revanche, en présence de milieu de transport selon l'invention, que ce soit après 1 ou 10 heures d'incubation (figure 2B et 2C, respectivement), les inventeurs n'ont observé la présence d'aucune colonie et donc d'aucun contaminant, mettant en avant le rôle décontaminant du milieu de transport et ce, dès la 1^{ère} heure d'incubation.

3.2) Les inventeurs ont observé que le milieu de transport selon l'invention permettait une très bonne survie de *Mycobacterium tuberculosis,* et était même favorable à sa croissance. En effet, après 16 jours d'incubation, les milieux ont été observés pour mesurer l'impact du milieu de transport sur la survie de *Mycobacterium tuberculosis* en comparaison à un milieu PBS (figures 3A à 3C).

Les inventeurs ont observé que le nombre de colonies était plus important lorsque le prélèvement a été pré-incubé dans le milieu de transport, et ce dès 2 heures d'incubation (figure 3B), par comparaison à un milieu PBS (figure 3A).
Fig.3A : Témoin négatif (milieu PBS).
Fig. 3B : Après 2 heures d'incubation dans le milieu de transport selon l'invention.
Fig. 3C : Après 10 heures d'incubation dans le milieu de transport selon l'invention.

Ces résultats permettent de conclure que le milieu de transport selon l'invention permet d'une part une décontamination efficace du prélèvement, rendant l'étape standard actuelle de décontamination par la soude inutile, et d'autre part réalise une pré-incubation ou amorce de culture de *Mycobacterium tuberculosis.*

3.3) Les inventeurs ont déterminé que la concentration de surnageant de BCG devait être de préférence dans une proportion d'au moins 2,5%.

Pour ce faire, 2 mL d'expectoration Ziehl négative ont été artificiellement infectés avec 200 µL d'une suspension bactérienne de mycobactéries calibrée à 10⁶ par densité optique. Ce prélèvement a ensuite été ajouté à 5 mL de milieu de transport contenant 10, 5, 2.5, 1.25, 0.625 et 0.3125% de surnageant *M. bovis* et incubé à 37°C pendant 10 heures. Toutes les heures, 100 µL de suspension ont été prélevés et ensemencés au râteau sur milieu COS.

Le témoin négatif, qui n'a pas subi d'étape de pré-incubation, a été réalisé avec 200 µL de suspension mycobactérienne calibrée par densité optique à 10⁶ mycobactéries/mL dilués dans 7 mL de PBS (volume correspondant au volume de milieu de transport + expectoration). 100 µL de suspension ont été prélevés et ensemencés au râteau sur milieu COS.

Les géloses ont été incubées dans une étuve à 37°C sous 5% CO₂.

Après 16 jours d'incubation, les milieux ont été observés pour mesurer l'impact du milieu de transport sur la survie de *Mycobacterium tuberculosis* selon la proportion en surnageant de BCG, en comparaison à un milieu PBS.

Les inventeurs ont observé qu'une proportion en surnageant d'au moins 2,5% était nécessaire pour conserver une bonne survie de *Mycobacterium tuberculosis* dans le milieu de transport, par comparaison à un milieu PBS.

3.4) Les inventeurs ont analysé l'impact du milieu de transport sur l'examen direct microscopique par la coloration de Ziehl témoignant de la viabilité de *Mycobacterium tuberculosis* après incubation d'un prélèvement d'expectoration dans le milieu de transport selon l'invention. Pour ce faire, 2 mL d'expectoration Ziehl négative ont été artificiellement infectés avec 200 µL d'une suspension bactérienne de mycobactéries calibrée à 10⁶ par densité optique. Ce prélèvement a ensuite été ajouté à 5 mL de milieu de transport et incubé à 37°C pendant 26 heures. Après 26 heures d'incubation, 200 µL de suspension ont été insérés dans une chambre à cytospin et centrifugés 5 minutes à 2000 tours. Après séchage de la lame, la coloration de Ziehl a été effectuée à l'aide d'un colorateur automatique (AFB Auto Stainer AT-2002, Biocentric, Bandol, France).

On observe des amas de bacilles BAAR sur les 2 lames réalisées après 26 heures d'incubation dans le milieu de transport selon l'invention, démontrant ainsi que la coloration de Ziehl peut être réalisée sur le prélèvement préalablement incubé dans le milieu de transport. Les inventeurs en ont conclu que le milieu de transport selon l'invention, malgré la présence de colorant (vert malachite) et de contaminants morts, ne constituait pas un obstacle à la coloration des mycobactéries.

### BIBLIOGRAPHIE

(1) Mignard S, Flandrois JP. A seven-gene, multilocus, genus-wide approach to the phylogeny of mycobacteria using supertrees. Int J Syst Evol Microbiol. 2008;58:1432-41.
(2) Pfyffer GE. Mycobacterium : general characteristics, laboratory detection, in staning procedures. In : Murray Pr, Baron EJ, Jorgensen JH, Landry ML, Pfaller MA. Manual of Clinical Microbiology 9ème Eds. Amercian Society for Microbiology, Washington DC ; 2007, pp. 543-572.
(3) Cole S et al. Massive gene decay in the leprosy bacillus. Nature 2001;409:1007-11.
(4) Asmar S & Drancourt M. Rapid culture-based diagnosis of pulmonary tuberculosis in developed and developing countries. Frontiers in Microbiology 2016 ; 7 :30.
(5) Dye C. et al. Prospects for worlwide tuberculosis control under the WHO DOTS strategy. Lancet 1998; 352:1886-91.
(6) World Health Organization. 2016. Global tuberculosis control : surveillance, planning, financing. WHO report. Geneva : World Health Organization.
(7) El Khéchine A., Henry M., Raoult D., Drancourt M. (2009). Detection of Mycobacterium tuberculosis complex organisms in the stools of patients with pulmonary tuberculosis. Microbiology. 155, 2384-9; Bonnave P.E., Raoult D., Drancourt M. (2013). Gastric aspiration is not necessary for the diagnosis of pulmonary tuberculosis. Eur J Clin Microbiol Infect Dis. 32, 569-71.
(8) Paramasivan C. N., Narayana A. S., Prabhakar R., Rajagopal M. S., Somasundaram P. R., Tripathy S. P. (1983). Effect of storage of sputum specimens at room temperature on smear and culture results. Tubercle 64, 119-124].
(9) Tessema B., Beer J., Emmrich F., Sack U., Rodloff A. C. (2011). Rate of recovery of Mycobacterium tuberculosis from frozen acid-fast-bacillus smear-positive sputum samples subjected to long-term storage in Northwest Ethiopia. J. Clin. Microbiol. 49, 2557-2561.
(10) Lumb R., Ardian M., Waramori G., Syahrial H., Tjitra E., Maguire G. P., et al. . (2006). An alternative method for sputum storage and transport for Mycobacterium tuberculosis drug resistance surveys. Int. J. Tuberc. Lung Dis. 10, 172-177; Palomino J. C. (2007). Tuberculosis 2007. From Basic Science to Patient Care, 1st Edn. Edited by: Palomino J. C., Leao S. C., Ritacco V., editors. , 93-105. Available online at: www.TuberculosisTextbook.com.
(11) Pardini M., Varaine F., Iona E., Arzumanian E., Checchi F., Oggioni M. R., et al. . (2005). Cetyl-pyridinium chloride is useful for isolation of Mycobacterium tuberculosis from sputa subjected to long-term storage. J. Clin. Microbiol. 43, 442-444
(12) Selvakumar N., Narayana A. S. (1993). Use of cetylpridinium chlorides for storage of sputum specimens and isolation of M. tuberculosis. J. Bacteriol. 191, 4714-4721;
(13) Rieder H. L., Rieder H. L. (1998). The Public Health Service National Tuberculosis Reference Laboratory and the National Laboratory Network: minimum requirements, role and operation in a low-income country. J. Bacteriol. 191, 4714-4721.
(14) Smithwick R. W., Stratigos C. B., David H. L. (1975). Use of cetylpyridinium chloride and sodium chloride for the decontamination of sputum specimens that are transported to the laboratory for the isolation of Mycobacterium tuberculosis. J. Clin. Microbiol. 1, 411-413.
(15) Peres P. J., Gevaudan M. J., Gulian P. C., de Micco P. (1988). Une méthode de traitement des produits pathologiques en vue de l'isolement des mycobactéries. Rev. Franç. Labo. 173, 67-74; Asmar S.,
(16) Asmar S., Drancourt M. (2015). Chlorhexidine decontamination of sputum for culturing Mycobacterium tuberculosis. BMC Microbiol. 15:155
(17) 12 Selvakumar N., Vanajakumar Gopi, P. G., Venkataramu K. V., Datta M., Paramasivan C. N., et al. . (1995). Isolation of tubercle bacilli from sputum samples of patients in the field studies by the cetylpyridinium chloride-sodium chloride & sodium hydroxide methods. Indian J. Med. Res. 102, 149-151;
(18) Bobadilla-del-Valle M., Ponce-de-Leôn A., Kato-Maeda M., Hernández-Cruz A., Calva-Mercado J. J., Chávez-Mazari B., et al.. (2003). Comparison of sodium carbonate, cetyl-pyridinium chloride, and sodium borate for preservation of sputa for culture of Mycobacterium tuberculosis. J. Clin. Microbiol. 41, 4487-4488.
(19) Pal N., Sharma B., Malhotra B., Rishi S. (2009). Transport and storage of sputum specimen by using cetylpyridinium chloride for isolation of mycobacteria. Indian J. Pathol. Microbiol. 52, 59-61].
(20) Ferroni A., Vu-Thien H., Lanotte P., Le Bourgeois M., Sermet-Gaudelus I., Fauroux B., et al. . (2006). Value of the chlorhexidine decontamination method for recovery of nontuberculous mycobacteria from sputum samples of patients with cystic fibrosis. J. Clin. Microbiol. 44, 2237-2239.
(21)Tessema B., Beer J., Emmrich F., Sack U., Rodloff A. C. (2011). Rate of recovery of Mycobacterium tuberculosis from frozen acid-fast-bacillus smear-positive sputum samples subjected to long-term storage in Northwest Ethiopia. J. Clin. Microbiol. 49, 2557-2561.
(22) Jena J., Panda B. N. (1999). Evaluation of modified trisodium phosphate transport medium and single step culture method for M. tuberculosis. Ind. J. Tuberc. 46, 197-200.
(23) Jena J., Panda B. N. (2004). Evaluation of trisodium phosphate as a transport medium and its utility in a single-step decontamination technique for the culture of M. tuberculosis. Ind. J. Tuberc. 51, 137-140;
(24) Chauhan M. M. (1999). Assessment of trisodium phosphate for storage and isolation of M. tuberculosis. J. Bacteriol. 191, 4714-4721.
(25) Asmar S & Drancourt M. Rapid culture-based diagnosis of pulmonary tuberculosis in developed and developing countries. Frontiers in Microbiology 2016 ; 6:1184.
(26) Ghodbane R, Raoult D, Drancourt M. Dramatic reduction of culture time of Mycobacterium tuberculosis. Sci Rep. 2014;4:4236.
(27) Ghodbane R., Asmar S., Betzner M., Linet M., Pierquin J., Drancourt M. (2015). Real-time video imaging for rapid detection of Mycobacterium tuberculosis micro-colonies. J Clin Microbiol. 53, 2693-6.

## Revendications

1. Milieu liquide de transport et/ou conservation contenant ou destiné à contenir un échantillon de mycobactéries du complexe *Mycobacterium tuberculosis* pour le transport et/ou la conservation de dites mycobactéries du complexe *Mycobacterium tuberculosis,* comprenant un milieu de culture de base de dites mycobactéries et, des composés antibiotiques sans activité à l'égard des mycobactéries, et au moins un agent décontaminant, de préférence la Chlorhexidine et le vert de malachite, **caractérisé en ce qu'**il comprend en outre une proportion d'au moins 2,5 % en volume de surnageant complet de culture de *Mycobacterium bovis* souche BCG comme composant additionnel ledit surnageant étant récolté avant la phase stationnaire de croissance après moins de deux semaines de culture.

2. Milieu selon la revendication 1, **caractérisé en ce que** le surnageant complet de culture de *Mycobacterium bovis* souche BCG est récolté après pas plus de 10 jours de culture.

3. Milieu selon l'une des revendications 1 à 2, **caractérisé en ce qu'**il s'agit d'un milieu de transport liquide comprenant en outre au moins un agent dispersant apte à disperser les agrégats de mycobactéries d'un échantillon clinique de mycobactéries, de préférence ledit agent dispersant étant un détergent tel que du tween 80.

4. Milieu selon la revendication 3, **caractérisé en ce que** ledit agent dispersant est un détergent tel que du tween 80, à une concentration d'au moins 0,01 % et inférieure à 1% dans le dit milieu, de préférence inférieure à 0.1%.

5. Milieu selon l'une des revendications 3 ou 4, **caractérisé en ce qu'**il comprend:
- la Chlorhexidine et le vert de malachite, et
- la lécithine d'œuf, et
- au moins une source de carbone, de préférence du glycérol, et
- des composés antibiotiques, de préférence la colimycine et la pénicilline et antifongique, l'amphotéricine.

6. Milieu selon l'une des revendications 1 à 5, **caractérisé en ce qu'**il comprend un milieu de culture de mycobactéries de base comprenant, outre de l'eau distillée, les composants suivants : sulfate d'ammonium, sulfate de magnésium, sulfate de cuivre, sulfate de zinc, citrate de sodium, citrate d'ammonium ferrique, chlorure de sodium, chlorure de calcium, phosphate monopotassique, phosphate disodique, acide L-glutamique, biotine et pyridoxine.

7. Milieu selon l'une des revendications 1 à 6, **caractérisé en ce qu'**il comprend desdits antibiotiques sans activité antimycobactérienne aux concentrations employées choisis parmi les colymicine et penicilline, et le milieu de culture comporte, en outre, un antifongique, de préférence l'amphotéricine B.

8. Milieu selon l'une des revendications 6 ou 7, **caractérisé en ce qu'**il comprend un milieu de culture de base de mycobactérie qui est un milieu liquide Middlebrook de référence 7H9.

9. Milieu selon la revendication 8, **caractérisé en ce qu'**il s'agit d'un milieu de transport comprenant les composants suivants, de préférence dans les quantités et teneurs pondérales ou volumiques suivantes pour 1L:
| | |
|---|---|
| - Middlebrook 7H9 | : 4,7g (0.47%) |
| - Glycérol | : 4 mL (0,4 %) |
| - Lécithine d'œuf | : 5g (0.5%) |
| - OADC | : 100mL (10%) |
| - Chlorhexidine solution 20% | : 15mL (1.5%) |
| - Tween 80 | : 0.5mL (0.05%) |
| - Vert malachite | : 0.25g (0.025%) |
| - Colimycine | : 0.05g (0.005%) |
| - Pénicilline G | : 2.5g (0.25%) |
| - Amphotéricine B | : 0.01g (0.001%) |
| - Surnageant *M. bovis* BCG | : 25mL (2.5 %) |

10. Procédé de transport et/ou conservation de mycobactérie, de préférence la bactérie *Mycobacterium tuberculosis,* dans un échantillon de prélèvement clinique en contenant, assurant la viabilité de mycobactérie pendant au moins 4 heures de préférence au moins 48 heures, **caractérisé en ce que** l'on réalise le transport et/ou conservation dans un dit milieu de transport et/ou conservation selon l'une des revendications 1 à 9.

11. Procédé selon la revendication 10 **caractérisé en ce qu'**on réalise le transport et puis la culture d'un échantillon de prélèvement clinique contenant ou susceptible de contenir des mycobactéries du complexe *Mycobacterium tuberculosis,* de préférence la bactérie *Mycobacterium tuberculosis,* dans lequel :
a) on réalise le transport et/ou la conservation dudit échantillon de prélèvement clinique dilué dans un milieu liquide de transport et conservation selon l'une des revendications 1 à 9 contenant des composés antibiotiques et au moins un composé antifongique, et
b) on réalise ensuite la culture des mycobactéries du dit échantillon de prélèvement clinique dilué dans un milieu de culture de dites mycobactéries ne contenant pas de composés antibiotiques et au moins un composé antifongique.

12. Procédé selon l'une des revendications 10 à 11 **caractérisé en ce que** le dit surnageant complet de culture est récolté avant la phase stationnaire de croissance de la bactérie après moins de 2 semaines de culture, de préférence encore pas plus de 10 jours de culture.

13. Procédé selon l'une des revendications 10 ou **caractérisé en ce que** le dit surnageant complet de culture est obtenu à partir d'une culture de *Mycobacterium bovis* souche BCG dans un premier milieu de culture présentant les mêmes composants que ledit milieu de culture de base de mycobactérie.

14. Procédé selon l'une des revendications 10 à 13 **caractérisé en ce qu'**à l'étape b), ledit milieu de culture dites mycobactéries comprend les mêmes composants que le dit milieu de transport à l'exception des dits agents décontaminants et en outre des facteurs de croissance de mycobactéries additionnels suivants : hydrolysat de caséine, protéose peptone, extrait de levure, extrait de bœuf, amidon de pommes de terre, glucose, sérum d'agneau inactivé, huile d'olive et au moins un composé antioxydant de préférence choisi parmi L-cystéine, acide urique, acide ascorbique et L-glutathion.

## Patentansprüche

1. Flüssiges Transport- und/oder Konservierungsmedium, das eine Probe von Mykobakterien des Komplexes *Mycobacterium-tuberculosis* enthält oder dazu bestimmt ist, diesen zu enthalten, für den Transport und/oder die Konservierung der Mykobakterien des Komplexes *Mycobacterium-tuberculosis,* umfassend ein Basiskulturmedium für die Mykobakterien und antibiotische Verbindungen ohne Aktivität gegenüber Mykobakterien und mindestens ein Dekontaminationsmittel, vorzugsweise Chlorhexidin und Malachitgrün, **dadurch gekennzeichnet, dass** es ferner einen Anteil von mindestens 2,5 Volumen- % an vollständigem Überstand der Kultur von *Mycobacterium bovis* Stamm BCG als zusätzliche Komponente umfasst, wobei der Überstand vor der stationären Wachstumsphase nach weniger als zwei Wochen Kultur geerntet wird.

2. Medium nach Anspruch 1, **dadurch gekennzeichnet, dass** der vollständige Kulturüberstand von *Mycobacterium bovis* Stamm BCG nach nicht mehr als 10 Tagen Kultur geerntet wird.

3. Medium nach einem der Ansprüche 1 bis 2, **dadurch gekennzeichnet, dass** es sich um ein flüssiges Transportmedium handelt, ferner umfassend mindestens ein Dispergiermittel, das geeignet ist, um die Mykobakterienaggregate einer klinischen Probe von Mykobakterien zu dispergieren, wobei das Dispergiermittel vorzugsweise ein Detergens ist, wie beispielsweise Tween 80.

4. Medium nach Anspruch 3, **dadurch gekennzeichnet, dass** das Dispergiermittel ein Detergens ist, wie beispielsweise Tween 80, in einer Konzentration von mindestens 0,01 % und weniger als 1 % in dem Medium, vorzugsweise weniger als 0,1 %.

5. Medium nach einem der Ansprüche 3 oder 4, **dadurch gekennzeichnet, dass** es Folgendes umfasst:
- Chlorhexidin und Malachitgrün, und
- Lecithin aus Eiern, und
- mindestens eine Kohlenstoffquelle, vorzugsweise Glycerin, und
- antibiotische Verbindungen, vorzugsweise Colimycin und Penicillin, und antimykotische Verbindungen, Amphotericin.

6. Medium nach einem der Ansprüche 1 bis 5, **dadurch gekennzeichnet, dass** es ein Mykobakterien-Basiskulturmedium umfasst, das außer dem destillierten Wasser die folgenden Komponenten umfasst: Ammoniumsulfat, Magnesiumsulfat, Kupfersulfat, Zinksulfat, Natriumcitrat, Eisenammoniumcitrat, Natriumchlorid, Calciumchlorid, Monokaliumphosphat, Dinatriumphosphat, L-Glutaminsäure, Biotin und Pyridoxin.

7. Medium nach einem der Ansprüche 1 bis 6, **dadurch gekennzeichnet, dass** es die Antibiotika ohne antimykobakterielle Aktivität in den verwendeten Konzentrationen umfasst, die ausgewählt sind aus Colymicin und Penicillin, und das Kulturmedium ferner ein Antimykotikum, vorzugsweise Amphotericin B, umfasst.

8. Medium nach einem der Ansprüche 6 oder 7, **dadurch gekennzeichnet, dass** es ein Mykobakterien-Basiskulturmedium umfasst, das ein Middlebrook-Medium mit der Nummer 7H9 ist.

9. Medium nach Anspruch 8, **dadurch gekennzeichnet, dass** es sich um ein Transportmedium handelt, umfassend die folgenden Komponenten, vorzugsweise in den folgenden Mengen und Gewichts- oder Volumengehalten pro 1 l:
- Middlebrook 7H9: 4,7 g (0,47 %)
- Glycerin: 4 ml (0,4 %)
- Ei-Lecithin: 5 g (0,5 %)
- OADC: 100 ml (10 %)
- Chlorhexidin-Lösung 20 %: 15 ml (1,5 %)
- Tween 80: 0,5 ml (0,05 %)
- Malachitgrün: 0,25 g (0,025 %)
- Colimycin: 0,05 g (0,005 %)
- Penicillin G: 2,5 g (0,25 %)
- Amphotericin B: 0,01 g (0,001 %)
- Überstand *M. bovis* BCG: 25 ml (2,5 %)

10. Verfahren zum Transport und/oder zur Konservierung von Mykobakterien, vorzugsweise des Bakteriums *Mycobacterium tuberculosis,* in einer klinischen Probe, die diese enthält, wobei die Lebensfähigkeit der Mykobakterien über mindestens 4 Stunden, vorzugsweise mindestens 48 Stunden, sichergestellt ist, **dadurch gekennzeichnet, dass** der Transport und/oder die Aufbewahrung in einem besagten Transport- und/oder Aufbewahrungsmedium nach einem der Ansprüche 1 bis 9 durchgeführt wird.

11. Verfahren nach Anspruch 10, **dadurch gekennzeichnet, dass** der Transport und dann die Kultivierung einer klinischen Probe durchgeführt wird, die Mykobakterien des Komplexes *Mycobacterium tuberculosis,* vorzugsweise das Bakterium *Mycobacterium tuberculosis,* enthält oder enthalten kann, wobei:
a) der Transport und/oder die Aufbewahrung der verdünnten klinischen Probe in einem flüssigen Transport- und Aufbewahrungsmedium nach einem der Ansprüche 1 bis 9 durchgeführt wird, das antibiotische Verbindungen und mindestens eine antimykotische Verbindung enthält, und
b) anschließend die Mykobakterien der klinischen Probe, die in einem Kulturmedium für die Mykobakterien verdünnt wurde, das keine antibiotischen Verbindungen und mindestens eine antimykotische Verbindung enthält, kultiviert werden.

12. Verfahren nach einem der Ansprüche 10 bis 11 **dadurch gekennzeichnet, dass** der vollständige Kulturüberstand vor der stationären Wachstumsphase des Bakteriums nach weniger als 2 Wochen Kultur, vorzugsweise noch nicht mehr als 10 Tagen Kultur, geerntet wird.

13. Verfahren nach einem der Ansprüche 10 oder **dadurch gekennzeichnet, dass** der vollständige Kulturüberstand aus einer Kultur von *Mycobacterium bovis* Stamm BCG in einem ersten Kulturmedium erlangt wird, das die gleichen Bestandteile wie das genannte Mycobacterium-Basiskulturmedium aufweist.

14. Verfahren nach einem der Ansprüche 10 bis 13 **dadurch gekennzeichnet, dass** in Schritt b) das Mykobakterien-Kulturmedium die gleichen Komponenten wie das Transportmedium mit Ausnahme von Dekontaminationsmitteln, und zusätzlich die folgenden zusätzlichen Mykobakterien-Wachstumsfaktoren umfasst: Caseinhydrolysat, Proteosepepton, Hefeextrakt, Rindfleischextrakt, Kartoffelstärke, Glucose, inaktiviertes Lammserum, Olivenöl und mindestens eine antioxidative Verbindung, die vorzugsweise ausgewählt ist aus L-Cystein, Harnsäure, Ascorbinsäure und L-Glutathion.

## Claims

1. Liquid medium for transport and/or preservation containing or intended to contain a sample of mycobacteria of the *Mycobacterium tuberculosis* complex for transport and/or preservation of said mycobacteria of the *Mycobacterium tuberculosis* complex, comprising a basic culture medium of said mycobacteria and antibiotic compounds without activity towards mycobacteria, and at least one decontaminating agent, preferably chlorhexidine and malachite green, **characterized in that** it further comprises a proportion of at least 2.5% by volume of complete culture supernatant of *Mycobacterium bovis* strain BCG as an additional component, said supernatant being harvested before the stationary growth phase after less than two weeks of culture.

2. Medium according to claim 1, **characterized in that** the complete culture supernatant of *Mycobacterium bovis* strain BCG is harvested after no more than 10 days of culture.

3. Medium according to one of claims 1 to 2, **characterized in that** it is a liquid transport medium further comprising at least one dispersing agent capable of dispersing the mycobacteria aggregates of a mycobacteria clinical sample, preferably said dispersing agent being a detergent such as tween 80.

4. Medium according to claim 3, **characterized in that** said dispersing agent is a detergent such as tween 80, at a concentration of at least 0.01% and less than 1% in said medium, preferably less than 0.1%.

5. Medium according to one of claims 3 or 4, **characterized in that** it comprises:
- Chlorhexidine and malachite green, and
- egg lecithin, and
- at least one carbon source, preferably glycerol, and
- antibiotic compounds, preferably colimycin and penicillin, and antifungal compounds, amphotericin.

6. Medium according to one of claims 1 to 5, **characterized in that** it comprises a basic mycobacteria culture medium comprising, in addition to distilled water, the following components: ammonium sulfate, magnesium sulfate, copper sulfate, zinc sulfate, sodium citrate, ferric ammonium citrate, sodium chloride, calcium chloride, monopotassium phosphate, disodium phosphate, L-glutamic acid, biotin and pyridoxine.

7. Medium according to one of claims 1 to 6, **characterized in that** it comprises said antibiotics without antimycobacterial activity at the concentrations employed selected from colymicin and penicillin, and the culture medium further comprises an antifungal agent, preferably amphotericin B.

8. Medium according to one of claims 6 or 7, **characterized in that** it comprises a basic mycobacterial culture medium which is a Middlebrook liquid medium of reference 7H9.

9. Medium according to claim 8, **characterized in that** it is a transport medium comprising the following components, preferably in the following quantities and contents by weight or by volume per 1L:
| | |
|---|---|
| - Middlebrook | : 4,7g (0.47%) |
| - Glycerol | : 4 mL (0.4%) |
| - Egg lecithin | : 5g (0.5%) |
| - OADC | : 100mL (10%) |
| - Chlorhexidine 20% solution | : 15mL (1.5%) |
| - Tween 80 | : 0.5mL (0.05%) |
| - Malachite green | : 0.25g (0.025%) |
| - Colimycin | : 0.05g (0.005%) |
| - Penicillin G | : 2.5g (0.25%) |
| - Amphotericin B | : 0.01g (0.001%) |
| - Supernatant *M*. *bovis* | : 25mL (2.5 %) |

10. A method of transporting and/or preserving mycobacteria, preferably the bacterium *Mycobacterium tuberculosis,* in a clinical sample containing them, ensuring the viability of mycobacteria for at least 4 hours, preferably at least 48 hours, **characterized in that** the transport and/or preservation is carried out in a said transport and/or preservation medium according to one of claims 1 to 9.

11. Method according to claim 10, **characterized in that** a clinical sample containing or liable to contain mycobacteria of the *Mycobacterium tuberculosis* complex, preferably the bacterium *Mycobacterium tuberculosis,* is transported and then cultured, in which :
a) said diluted clinical sample is transported and/or preserved in a liquid transport and preservation medium according to one of claims 1 to 9 containing antibiotic compounds and at least one antifungal compound, and
b) mycobacteria are then cultured from said clinical sample diluted in a culture medium for said mycobacteria containing no antibiotic compounds and at least one antifungal compound.

12. Method according to one of claims 10 to 11, **characterized in that** said complete culture supernatant is harvested before the stationary phase of bacterial growth after less than 2 weeks of culture, preferably not more than 10 days of culture.

13. Method according to one of claims 10 or **characterized in that** said complete culture supernatant is obtained from a culture of *Mycobacterium bovis* strain BCG in a first culture medium having the same components as said basic mycobacterial culture medium.

14. Method according to one of claims 10 to 13, **characterized in that** in step b), said mycobacteria culture medium comprises the same components as said transport medium with the exception of said decontaminating agents and furthermore the following additional mycobacteria growth factors: casein hydrolysate, peptone proteose, yeast extract, beef extract, potato starch, glucose, inactivated lamb serum, olive oil and at least one antioxidant compound preferably selected from L-cysteine, uric acid, ascorbic acid and L-glutathione.
